# EUROPÄISCHE PATENTSCHRIFT

(11) **EP 0 920 341 B1**
(45) Veröffentlichungstag und Bekanntmachung des Hinweises auf die Patenterteilung: **12.03.2003**
(21) Anmeldenummer: 97937583.9
(22) Anmeldetag: 13.08.1997
(51) Int. Cl.: A61L 24/00, A61L 27/00

(54) **VERFAHREN ZUR HERSTELLUNG VON WIRKSTOFFHALTIGEN KNOCHENZEMENTEN**
PROCESS FOR PRODUCING BONE CEMENT CONTAINING ACTIVE SUBSTANCES
PROCEDE POUR LA FABRICATION DE CIMENTS OSSEUX CONTENANT DES PRINCIPES ACTIFS

(30) Priorität: 22.08.1996 DE 19641775
(43) Veröffentlichungstag der Anmeldung: 09.06.1999
(73) Patentinhaber: MERCK PATENT GmbH, 64293 Darmstadt (DE)
(72) Erfinder: NIES, Berthold, D-64407 Fränkisch-Crumbach (DE)
(86) Internationale Anmeldenummer: EP9704434
(87) Internationale Veröffentlichungsnummer: WO98007456

(56) Entgegenhaltungen:
- EP-A- 0 701 824
- EP-A- 0 705 609

## Beschreibung

Die Erfindung betrifft ein Verfahren zur Herstellung von wirkstoffhaitigen Knochenzementen, sowie daraus herstellbarer Knochenersatzmaterialien oder implantierbarer Pharmakadepots.

Knochenzemente, Knochenersatzmaterialien und implantierbare Pharmakadepots, die auf Acrylatkunststofen basieren, sind seit langem bekannt. Polymermaterialien auf Basis von Acryl- und/oder Methacrylsäureestern haben sich hier aufgrund ihrer Biokompatibilität, ihrer vorzüglichen Festigkeitseigenschaften, ihrer günstigen Eigenschaften bei der Freisetzung eingelagerter pharmazeutischer Wirkstoffe und nicht zuletzt aufgrund ihrer anwendungsgerechten Verarbeitbarkeit bewährt.

Gängige Knochenzemente setzen sich aus etwa 50 bis 75 Gew.% einer Feststoffkomponente, die aus einem feinteiligen Polymerisat von Acrylund/oder Methacrylsäureestern sowie gegebenenfalls weiteren Zusätzen wie Polymerisationskatalysatoren, Röntgenkontrastmitteln, Füllstoffen und Farbstoffen besteht, und aus etwa 25 bis 50 Gew.% einer flüssigen Komponente, die aus einem Acryl- und/oder Methacrylsäureestermonomeren, sowie gegebenenfalls weiteren Zusätzen wie Polymerisationsbeschleunigem und Stabilisatoren besteht, zusammen. Zum Gebrauch werden Feststoffkomponente und flüssige Komponente zu einer flüssigen bis halbfesten Paste vermengt, diese gegebenenfalls in eine gewünschte Form gebracht oder zur Einzementierung einer Prothese an dem Implantationsort appliziert. Die Aushärtung der Masse erfolgt durch die mit dem Vermischen der Komponenten induzierte Polymerisationsreaktion.

Sehr gebräuchlich ist zum Beispiel ein Knochenzement, der in einer Normalpackung 2 Beutel mit je etwa 40 g Polymerpulver und 2 Ampullen mit je 20 ml Monomerflüssigkeit enthält. Das Pulver ist ein feines Perlpolymerisat aus Methacrylsäuremethylester mit einem Copolymeranteil von Acrylsäuremethylester. Als Katalysator sind dem Pulver etwa 0,5 % Dibenzoylperoxid zugesetzt. Zur Kenzeichnung des Materials sind bei der Herstellung geringe Mengen von Chlorophyll miteinpolymerisiert. Das Pulver enthält zusätzlich ein übliches Röntgenkontrastmittel wie zum Beispiel Zirkondioxid. Die zugehörige Flüssigkeit besteht aus monomerem Methacrylsäuremethylester, dem als Polymerisationsbeschleuniger etwa 0,7 % Dimethyl-p-toluidin sowie als Stabilisator geringe Mengen Hydrochinon zugesetzt sind. Auch diese Flüssigkeit ist in der Regel zur Kennzeichnung mit einer geringen Menge Chlorophyll eingefärbt. Das in Polyethylenbeutel abgepackte Pulver ist mit Ethylenoxid sterilisiert. Die Flüssigkeit ist sterilfiltriert und in Glasampullen abgefüllt.

Beim Zusammenmischen von 2 Gewichtsteilen Pulver mit einem Gewichtsteil Flüssigkeit reagiert das Dibenzoylperoxid mit dem Dimethyl-p-toluidin in der Flüssigkeit, wodurch die radikalische Polymerisation angeregt wird. Die Mischung ist so abgestimmt, daß sie schon nach etwa einer Minute als teigige Paste verwendet werden kann. Diese Paste bleibt für mehrere Minuten knetbar und beginnt dann unter Wärmeentwicklung auszuhärten. Nach etwa 5 bis 10 Minuten ist die Polymerisation im wesentlichen abgeschlossen. Während der Polymerisationsphase, solange die Paste noch formbar ist, kann diese in jede gewünschte Form gebracht werden, also zum Beispiel zum Ausfüllen von Knochenhöhlen oder zum Einzementieren von Prothesen direkt in den Körper gebracht oder zur Herstellung von Formkörpem verwendet werden, die extrakorporal aushärten und danach an beliebigen Körperstellen eingesetzt werden können.

Bei zahlreichen Indikationen ist es wünschenswert, daß der Knochenzement pharmazeutische Wirkstoffe enthält. So können cytostatikahaltige Knochenzemente bei der Sanierung von Knochendefekten nach Entfernung von Knochentumoren eingesetzt werden. Bei der Einzementierung von Prothesen und bei der Osteosynthese sind Knochenzemente, die Antibiotika, Antiseptika sowie gegebenenfalls knochenwachstumsfördernde Substanzen enthalten, vorteilhaft. Formkörper aus wirkstoffhaltigen Knochenzementen können in Weichteilgewebe als lokale Wirkstoffdepots mit verzögerter Wirkstofffreisetzung implantiert werden.
Handelsübliche Knochenzemente mit Antibiotikazusatz (z.B. Septopal®) werden gelegentlich auch als Wirkstoffträger zur Behandlung lokaler Infektionen genutzt. Zusätzlich werden solchen Zementen weitere Antibiotika zugesetzt, um die Wirksamkeit zu erhöhen.

In EP 0 202 445 A1 ist beispielsweise ein derartiger cytostatikahaltiger Knochenzement sowie ein daraus hergestelltes Pharmakadepot mit besonders günstigen Freisetzungseigenschaften beschrieben. Aus dieser Druckschrift geht hervor, daß der jeweilige Wirkstoff dem Basismaterial des Knochenzementes, also dem Präpolymeren und/oder dem Monomeren als feinteiliges Pulver, zugemischt wird, so daß es dann in dem entstehenden Polymerisat homogen verteilt vorliegt.

In EP 0 701 824 A2 wird ein Verfahren zur Herstellung von wirkstoffhaltigen Knochenzementen beschrieben, wobei der Wirkstoff in einem organischen Lösungsmittel aufgelöst wird, und diese Lösung mit der flüssigen Komponente oder mit der Feststoffkomponente vermischt wird. Auf diese Weise können alle drei Komponenten des Knochenzementes in steriler Form zur Verfügung gestellt werden.

Die Beimischung freisetzungsfördernder Substanzen als Pulver zum Zement ist an sich bekannt. So wird z.B. in DE 26 51 441 C2 der Zusatz von Aminosäuren beschrieben.

Die Knochenzementkomponenten in vorkonfektionierter Form, die in erster Linie auf ihre mechanischen Eigenschaften oder auf eine einfache Weise der Sterilisierung der Komponenten hin optimiert sind, entsprechen jedoch nicht den Anforderungen, die an ein für die Implantation in den Körper vorgesehenes medizinisches Produkt gestellt werden müssen, da sie für die Verwendung als Wirkstoffträger nur suboptimale Eigenschaften aufweisen.

Die Handzumischung, wie auch die Beimischung freisetzungsfördernder Substanzen als Pulver zum Zement (z.B. Salz, Zucker, Aminosäuren) hat den Nachteil, daß eine homogene und reproduzierbare Mischung praktisch nicht zu erreichen ist. Eine inhomogene Mischung erfordert notwendigerweise große Zuschlagmengen an Wirk- und Hilfsstoffen, um die gewünschten Freisetzungen zu erreichen. Bei der Verwendung handelsüblicher wirkstoffhaltiger Knochenzemente bleibt die Freisetzung meist deutlich unterhalb therapeutisch wünschenswerter Spiegel. Insbesondere gilt dies bei Implantaten mit ungünstigen geometrischen Verhältnissen (z.B. Zementplomben).

Es bestand daher die Aufgabe ein Verfahren zu entwickeln, mit dem wirkstoffhaltige Knochenzemente bzw. deren Vor- sowie auch ihre Folgeprodukte auf einfache Weise bereitgestellt werden können, bei denen die Kinetik der Wirkstofffreigabe im Vordergrund steht, d.h. eine verbesserte Freisetzung des Wirkstoffs erreicht wird,

Es wurde nun gefunden, daß durch Zumischung von geeigneten Lösungsmitteln zur Monomerkomponente von Knochenzementen die geschilderten Nachteile vermieden werden und sich aus wirkstoffbeladenen Zementen sehr effektive implantierbare Wirkstoffträger herstellen lassen. Es wird eine signifikant höhere Freisetzung des Wirkstoffs erreicht, die Präparation des Knochenzements ist einfach und es werden geringe Wirkstoffmengen benötigt.

Gegenstand der Erfindung ist somit ein Verfahren zur Herstellung von einem einen oder mehrere Wirkstoffe enthaltenden Knochenzement, Knochenersatzmaterial oder entsprechenden implantierbaren Pharmakadepot auf Basis von Acryl- und/oder Methacrylsäureesterpolymerisaten durch Vermischung einer
festen Komponente (i), im wesentlichen bestehend aus polymerem Acryl- und/oder Methacrylsäureester in einem bezogen zum Knochenzement vorliegenden Anteil von 50 bis 75 Gew.% und einem oder mehreren pharmazeutischen Wirkstoffen mit einer
flüssigen Komponente (ii), im wesentlichen bestehend aus monomerem Acryl- und/oder Methacrylsäureester in einem Anteil von 25 bis 50 Gew.%,
zu einer flüssigen bis halbfesten polymerisierbaren Paste, sowie gegebenenfalls Formgebung und Aushärtung derselben, dadurch gekennzeichnet, daß man die flüssige Komponente (ii) mit einem organischen Lösungsmittel, dessen Anteil 50 Gew.%, bezogen auf (ii), nicht übersteigt, auflöst und diese Lösung mit der festen Komponente (i) vermischt.

Bei dem erfindungsgemäßen Verfahren lassen sich alle üblichen Knochenzemente auf Acrylat-/Methacrylat-Basis bzw. die hierfür gebräuchlichen Ausgangsprodukte verwenden. Knochenzemente dieser Art sind im Handel erhältlich. Ihre Zusammensetzung und die Art ihrer Verarbeitung sind dem Fachmann geläufig.

Zur Herstellung eines wirkstoffhaltigen Knochenzementes ist es erfindungsgemäß vorgesehen, die Monomerkomponente in einem organischen Lösungsmittel aufzulösen und dann diese Monomerlösung mit der feinteiligen polymeren Feststoffkomponente des Knochenzementes, die den Wirkstoff enthält, zu vermischen.
Bei der Zementanmischung ist das Lösungsmittel nicht an der Reaktion beteiligt oder nimmt als Polymerisationspartner teil, wie im Fall von Vinylpyrrolidon.
Die Monomerlösung wird zuvor sterilfiltriert, die Feststoffkomponente wird einer Endsterilisation mittels Strahlung und/oder Ethylenoxid unterzogen.

Für die Bereitstellung der Monomerlösung sind im wesentlichen alle üblichen organischen Lösungsmittel geeignet.
Bevorzugte Lösungsmittel sind z.B. 2-Pyrrolidon, N-Methylpyrrolidon, Dimethylsulfoxid (DMSO), Tetrahydrofuran (THF), Dioxan, Ethylenglycol, Propandiol, Vinylpyrrolidon oder Mischungen der genannten Lösungsmittel.
Zweckmäßigerweise werden solche Lösungsmittel ausgewählt, in denen sich das vorgesehene Monomer gut löst. Die Menge an Lösungsmittel wird so gewählt, daß sie 50 Gew.%, bezogen auf das flüssige Monomer, nicht übersteigt. Hierdurch ist gewährleistet, daß sich bei der Mischung der Komponenten zum gebrauchsfertigen Knochenzement die Verarbeitungseigenschaften, die Aushärtungscharakteristik und die mechanische Festigkeit des ausgehärteten Knochenzementes nicht zu stark verändern.
Vorzugsweise wird eine solche Menge an Lösungsmittel gewählt, daß deren Anteil 1 bis 25 Gew.%, insbesondere 5 bis 20 Gew.%, bezogen auf die flüssige Monomerkomponente, beträgt.

Die zum Einsatz gelangende Menge an pharmazeutischem Wirkstoff, der dem Knochenzement beigemischt ist, ist abhängig von dessen spezifischer Wirksamkeit, der medizinischen Indikation und vom jeweiligen Anforderungsprofil des Knochenzementes bzw. des daraus herzustellenden Knochenersatzmaterials oder Pharmakadepots. In aller Regel ist ein Anteil an pharmazeutischem Wirkstoff von 0,1 bis 5 Gew.%, bezogen auf die Gesamtmenge an Knochenzement, ausreichend; in Einzelfällen, insbesondere bei der Herstellung von implantierbaren Pharmakadepots, kann der Wirkstoffanteil auch höher, etwa bis zu 40 Gew.% liegen.

Als Wirkstoffe kommen vorzugsweise Cytostatika wie Methotrexat, Cisplatin, Cyclophosphamid, Fluoruracil, Doxorubicin etc., Antibiotika wie Vancomycin, Netilmicin, Gentamicin, Clindamycin, Vancomycin, Teicoplanin etc., weiterhin Antiseptika sowie knochenwachstumsfördemde Substanzen in Betracht.

Überraschend hat sich gezeigt, daß das Freisetzungsverhalten von z.B. Antibiotikahaltigem Knochenzement, der nach dem erfindungsgemäßen Verfahren hergestellt worden ist, erheblich besser ist, als wenn die Monomerkomponente wie bisher direkt der Feststoffkomponente zugemischt wird.

Selbstverständlich kann das Freisetzungsverhalten des Wirkstoffes durch die bekannten und üblichen Zusätze noch beeinflußt und gegebenenfalls weiter verbessert werden. Als derartige Zusätze kommen Aminosäuren wie Arginin sowie Hydroxylapatit oder Natriumhydrogencarbonat, möglichst in feinteiliger Form mit Partikelgrößen unter 100 µm, in Frage. Durch solche Zusätze läßt sich insbesondere die Anfangskonzentration der Wirkstofffreisetzung regeln.

Die Feststoffkomponente, die üblicherweise als Perlpolymerisat von Methylmethacrylat-Methylacrylat-Copolymer mit Partikelgrößen zwischen 5 und 250 µm vorliegt, enthält einen Polymerisationskatalysator wie etwa Dibenzoylperoxid sowie den Pharmakawirkstoff. Weiterhin kann sie Röntgenkontrastmittel wie zum Beispiel Zirkondioxid, Farbstoffe zur KennZeichnung wie etwa Chlorophyll sowie Füllstoffe und gegebenenfalls weitere Zusätze enthalten. Gängige Füllstoffe sind beispielsweise osteoinduktiv bzw. osteokonduktiv wirkende Calciumphosphate wie insbesondere Hydroxylapatit und Tricalciumphosphat. Der Anteil aller dieser Zusätze kann in einem weiten Bereich variieren und ist abhängig vom jeweiligen Anforderungsprofil des Knochenzementes bzw. der entsprechenden Folgeprodukte. Er übersteigt in der Regel kaum 30 Gew.%, bezogen auf die Feststoffkomponente. Die flüssige Monomerkomponente Methylmethacrylat enthält in der Regel einen Polymerisationsbeschleuniger wie Dimethyl-p-toluidin sowie Hydrochinon als Stabilisator in den hierfür üblichen Mengen. Weiterhin können Farbstoffe und sonstige zweckmäßige Zusätze vorhanden sein. Die Feststoffkomponente läßt sich ohne weiteres mit γ-Strahlung oder mit Ethylenoxid sterilisieren; die flüssige Komponente kann einer Sterilfiltration unterzogen werden. Beide Komponenten können getrennt und steril in entsprechende Behältnisse abgefüllt werden.

Der wirkstoffhaltige Knochenzement wird zweckmäßigerweise in Form eines Sets bereitgestellt, das sich aus getrennten Packungen der zwei Hauptkomonenten zusammensetzt. Komponente (a) beinhaltet die Feststoffkomponente, bestehend aus einem feinteiligen Polymerisat von Acrylund/oder Methacrylsäureestern, einem pharmazeutischen Wirkstoff, sowie gegebenenfalls weiteren Zusätzen wie Polymerisationskatalysatoren, Röntgenkontrastmitteln, Füllstoffen und Farbstoffen, deren Anteil etwa 50 bis 75 Gew.% des Knochenzementes beträgt. Komponente (b) beinhaltet die flüssige Komponente, bestehend aus einem Acryl- und/oder Methacrylsäuremonomeren sowie gegebenenfalls weiteren Zusätzen wie Polymerisationsbeschleunigern, Stabilisatoren und freisetzungsfördernder Substanzen, deren Anteil etwa 25 bis 50 Gew.% des Knochenzementes beträgt, in einem organischen Lösungsmittel, dessen Anteil 50 Gew.%, bezogen auf die flüssige Komponente, nicht übersteigt,

Vorzugsweise sind die Mengen der Komponenten so aufeinander abgestimmt. daß die gesamten zwei Packungsinhalte miteinander vereinigt werden. Die Mengenabstimmung erfolgt nach Maßgabe des vorgesehenen Anwendungszweckes und je nach dem, ob ein niedrigviskoser, ein mittelviskoser oder ein hochviskoser Zement erwünscht ist. Die Feststoffkomponente ist dabei einer Endsterilisation mittels Strahlung oder Ethylenoxid, die flüssige Monomerlösung einer Sterilfiltration unterzogen worden und steril in geeignete Packmittel abgefüllt.

Zweckmäßig ist die Ergänzung dieses Sets mit einer Vorrichtung zum Anmischen und/oder Ausbringen des Knochenzementes. Entsprechende Vorrichtungen sind bekannt und gebräuchlich. Vorzugsweise ermöglichen entsprechende Vorrichtungen das Anmischen des Knochenzementes unter Vakuum sowie das kombinierte Ausbringen des Zementes mittels einer Knochenzementspritze.

Die Herstellung des gebrauchsfertigen wirkstoffhattigen Knochenzementes und seine weitere Verarbeitung erfolgen in völliger Analogie zu bisherigen Knochenzementsystemen.
Der wirkstoffhaltige Knochenzement kann in üblicher Weise während des flüssigen bzw. plastischen Stadiums für die Implantation von Knochenprothesen verwendet werden. Der Chirurg kann auch die Masse zu Formkörpem beliebiger Form und Größe verarbeiten und diese nach der Aushärtung als lokale Wirkstoffdepots in die zu behandelnden Körperbereiche implantieren. Derartige implantierbare Pharmakadepots können auch bereits vorgefertigt angeboten werden.

### Beispiel 1

Ein Knochenzement, der in 40 g Osteopal®-Zementpulver (bestehend aus Polymethylmethacrylat, Chlorophyll, Benzoylperoxid und Zirkondioxid) 2 g Vancomycin als Pulver enthält, wird mit 20 ml Methylmethacrylat-Monomer angerührt.
Entsprechend werden 3 weitere Proben mit Methylmethacrylat-Monomer hergestellt, dem 0,2, 1,0 bzw. 4 ml (1, 5 bzw. 20 Gew.%) Propandiol zugesetzt wurden.
Der dünnflüssige Zementteig wird in eine zweiteilige Metallform gegeben, die kugelförmige Ausnehmungen mit 7 mm Durchmesser aufweist. Nach Aushärtung des Zements werden die Kugeln entnommen und in vitro die Freisetzung des Wirkstoffs nach einem standardisierten Verfahren bestimmt.

Tabelle 1 zeigt die tägliche Vancomycin-Freisetzung in µg/ml aus den jeweiligen Knochenzementkugeln. Diese steigt bei Zusatz von 1 ml (5 Gew.%) Propandiol zum Monomer um den Faktor 5 in der Spitze (1.Tag) an. Auch zu späteren Zeitpunkten bleibt die Freisetzung ca. 3 bis 5mal so hoch wie mit dem Ausgangszement.

**Tabelle 1**

| Freisetzung von Vancomycin [µg/ml] | | | | |
|---|---|---|---|---|
| Tag | 0 Gew.% Propandiol | 1 Gew.% Propandiol | 5 Gew.% Propandiol | 20 Gew.% Propandiol |
| 0,1 | 25,88 | 34,69 | 74,70 | 70,20 |
| 1 | 47,06 | 74,17 | 233,61 | 238,63 |
| 2 | 25,75 | 35,45 | 86,03 | 129,44 |
| 3 | 18,49 | 24,84 | 59,11 | 100,47 |
| 4 | 14,49 | 26,30 | 51,24 | 86,21 |
| 5 | 14,87 | 29,68 | 47,00 | 79,06 |
| 6 | 17,33 | 28,25 | 57,27 | 80,59 |
| 7 | 11,78 | 21,53 | 48,86 | 56,06 |
| 8 | 9,78 | 20,50 | 37,78 | 47,01 |
| 9 | 10,41 | 17,30 | 23,89 | 41,64 |
| 10 | 8,86 | 12,97 | 23,76 | 33,54 |
| 14 | 5,19 | 9,71 | 15,13 | 23,89 |
| 28 | 2,26 | 3,36 | 5,46 | 21,42 |
| 84 | 0,79 | 1,09 | 2,78 | 4,31 |

### Beispiel 2

Ein Knochenzement, der in 40 g Osteopal-Zementpulver 2 g Netilmicin als Pulver enthält, wird mit 20 ml Methylmethacrylat-Monomer angerührt.
Entsprechend werden 3 weitere Proben mit Methylmethacrylat-Monomer hergestellt, dem 0,2, 1,0 bzw. 4 ml (1, 5 bzw. 20 Gew.%) Propandiol zugesetzt wurden.
Der dünnflüssige Zementteig wird in eine zweiteilige Metallform gegeben, die kugelförmige Ausnehmungen mit 7 mm Durchmesser aufweist. Nach Aushärtung des Zements werden die Kugeln entnommen und in vitro die Freisetzung des Wirkstoffs nach einem standardisierten Verfahren bestimmt.
Tabelle 2 zeigt die tägliche Netilmicin-Freisetzung in µg/ml aus den jeweiligen Knochenzementkugeln. Die Netilmicin-Freisetzung ist schon im Ausgangszement in der Spitze relativ hoch, fällt dann aber schnell ab. Ein Zusatz von 1 ml Propandiol (5 Gew.%) verdoppelt den Spitzenwert (1.Tag).
In den nachfolgenden Tagen, bis zum 9. Tag, vergrößert sich der Unterschied bis auf einen Faktor von ca. 10 und bleibt dann nahezu konstant.

**Tabelle 2**

| Freisetzung von Netilmicin [µg/ml] | | | | |
|---|---|---|---|---|
| Tag | 0 Gew.% Propandiol | 1 Gew.% Propandiol | 5 Gew.% Propandiol | 20 Gew.% Propandiol |
| 0,1 | 100,99 | 285,95 | 282,95 | 308,77 |
| 1 | 335,62 | 498,29 | 634,96 | 783,22 |
| 2 | 52,05 | 60,06 | 142,97 | 248,30 |
| 3 | 34,96 | 32,55 | 82,82 | 170,64 |
| 4 | 23,16 | 21,00 | 96,48 | 150,83 |
| 5 | 29,16 | 22,62 | 64,50 | 125,63 |
| 6 | 15,83 | 26,94 | 121,44 | 113,52 |
| 7 | 17,64 | 10,53 | 125,40 | 114,85 |
| 8 | 9,45 | 5,06 | 88,14 | 92,98 |
| 9 | 5,53 | 4,59 | 58,96 | 50,36 |
| 10 | 4,45 | 3,30 | 50,91 | 42,47 |
| 14 | 3,24 | 1,74 | 35,09 | 23,04 |
| 28 | 1,69 | 1,05 | 15,97 | 5,42 |
| 84 | 0,43 | 0,23 | 3,76 | 1,14 |
| 168 | 0,22 | 0,20 | 2,36 | 0,47 |

## Patentansprüche

1. Verfahren zur Herstellung von einem einen oder mehrere Wirkstoffe enthaltenden Knochenzement, Knochenersatzmaterial oder entsprechenden implantierbaren Pharmakadepot auf Basis von Acryl- und/oder Methacrylsäureesterpolymerisaten durch Vermischung einer
festen Komponente (i), im wesentlichen bestehend aus polymerem Acryl- und/oder Methacrylsäureester in einem bezogen zum Knochenzement vorliegenden Anteil von 50 bis 75 Gew.% und einem oder mehreren pharmazeutischen Wirkstoffen mit einer
flüssigen Komponente (ii), im wesentlichen bestehend aus monomerem Acryl- und/oder Methacrylsäureester in einem Anteil von 25 bis 50 Gew.%,
zu einer flüssigen bis halbfesten polymerisierbaren Paste, sowie gegebenenfalls Formgebung und Aushärtung derselben, **dadurch gekennzeichnet, daß** man die flüssige Komponente (ii) mit einem organischen Lösungsmittel, dessen Anteil 50 Gew.%, bezogen auf (ii), nicht übersteigt, auflöst und diese Lösung mit der festen Komponente (i) vermischt.

2. Verfahren nach Anspruch 1, **dadurch gekennzeichnet, daß** der Anteil an Lösungsmittel 1 bis 25 Gew. %, vorzugsweise 5 bis 20 Gew.%, bezogen auf die flüssige Komponente, beträgt.

3. Verfahren nach den Ansprüchen 1 oder 2, **dadurch gekennzeichnet, daß** die Feststoffkomponente einer Endsterilisation mittels Strahlung undloder Ethylenoxid unterzogen und die flüssige Komponente sterilfiltriert werden.

4. Verfahren nach den Ansprüchen 1 bis 3, **dadurch gekennzeichnet, daß** als Lösungsmittel 2-Pyrrolidon, N-Methylpyrrolidon, Vinylpyrrolidon, Dimethylsulfoxid, Tetrahydrofuran, Dioxan, Ethylenglycol, Propandiol oder Kombinationen hiervon verwendet werden.

5. Set zur Herstellung eines wirkstofffreisetzungsfördernden Knochenzementes. bestehend aus getrennten Packungen von
(a) einer Feststoffkomponente, deren Anteil etwa 50 bis 75 Gew.% des Knochenzementes beträgt, bestehend im wesentlichen aus einem feinteiligen Polymerisat von Acryl- und/oder Methacrylsäureestern und einem pharmazeutischen Wirkstoff, und
(b) einer Lösung einer flüssigen Komponente, deren Anteil etwa 25 bis 50 Gew.% des Knochenzementes beträgt, bestehend im wesentlichen aus einem Acryl- und/oder Methacrylsäuremonomeren, in einem organischen Lösungsmittel, dessen Anteil 50 Gew.%, bezogen auf die flüssige Komponente, nicht übersteigt.

6. Set nach Anspruch 5, **dadurch gekennzeichnet, daß** es eine Vorrichtung zum Anmischen und/oder Ausbringen des Knochenzementes enthält.

7. Set nach Anspruch 5 oder 6, **dadurch gekennzeichnet, daß** die Pakkungseinheit (a) einer Endsterilisation mittels Strahlung und/oder Ethylenoxid und der Inhalt der Packungseinheit (b) einer Sterilfiltration unterzogen worden ist.

## Claims

1. Process for the preparation of a bone cement, bone replacement material or corresponding implantable pharmaceutical depot based on acrylate and/or methacrylate polymers which comprises one or more active ingredients, by mixing a
solid component (i), essentially consisting of polymeric acrylates and/or methacrylates in a proportion, based on the bone cement, of from 50 to 75% by weight and one or more pharmaceutical active ingredients with a
liquid component (ii), essentially consisting of monomeric acrylates and/or methacrylates in a proportion of from 25 to 50% by weight,
to give a liquid to semi-solid polymerisable paste, and, if desired, shaping and curing thereof, **characterised in that** the liquid component (ii) is dissolved with an organic solvent, whose proportion does not exceed 50% by weight, based on (ii), and this solution is mixed with the solid component (i).

2. Process according to Claim 1, **characterised in that** the proportion of solvent is from 1 to 25% by weight, preferably from 5 to 20% by weight, based on the liquid component.

3. Process according to Claims 1 or 2, **characterised in that** the solid component is subjected to final sterilisation by means of radiation and/or ethylene oxide, and the liquid component is sterile-filtered.

4. Process according to Claims 1 to 3, **characterised in that** the solvent used is 2-pyrrolidone, N-methylpyrrolidone, vinylpyrrolidone, dimethyl sulfoxide, tetrahydrofuran, dioxane, ethylene glycol, propanediol or combinations thereof.

5. Set for the preparation of a bone cement which promotes the release of active ingredient, consisting of separate packs of
(a) a solid component, whose proportion is from about 50 to 75% by weight of the bone cement, essentially consisting of a finely divided polymer of acrylates and/or methacrylates and a pharmaceutical active ingredient, and
(b) a solution of the liquid component, whose proportion is from about 25 to 50% by weight of the bone cement, essentially consisting of an acrylic and/or methacrylic acid monomer, in an organic solvent, whose proportion does not exceed 50% by weight, based on the liquid component.

6. Set according to Claim 5, **characterised in that** it contains a device for mixing and/or applying the bone cement.

7. Set according to Claim 5 or 6, **characterised in that** pack unit (a) has been subjected to final sterilisation by means of radiation and/or ethylene oxide, and the contents of pack unit (b) have been subjected to sterile filtration.

## Revendications

1. Procédé pour la préparation d'un ciment osseux, d'un matériau de prothèse osseuse ou d'un dépôt de produit pharmaceutique implantable correspondant, contenant une ou plusieurs substances actives, à base de polymères d'esters d'acide acrylique et/ou d'acide méthacrylique, par mélange
d'un composant solide (i) essentiellement constitué d'ester d'acide acrylique et/ou d'acide méthacrylique polymère en une proportion présente, par rapport au ciment osseux, de 50 à 75 % en poids, et d'une ou plusieurs substances actives pharmaceutiques, avec
un composant liquide (ii) essentiellement constitué d'ester d'acide acrylique et/ou d'acide méthacrylique monomère en une proportion de 25 à 50 % en poids,
en une pâte polymérisable liquide à semi-solide, ainsi qu'éventuellement mise en forme et durcissement de celle-ci, **caractérisé en ce qu'**on dissout le composant liquide (ii) avec un solvant organique, dont la proportion n'excède pas 50 % en poids, par rapport à (ii), et on mélange cette solution avec le composant solide (i).

2. Procédé selon la revendication 1, **caractérisé en ce que** la proportion du solvant va de 1 à 25 % en poids, de préférence de 5 à 20 % en poids, par rapport au composant liquide.

3. Procédé selon la revendication 1 ou 2, **caractérisé en ce que** le composant solide est soumis à une stérilisation finale au moyen d'irradiation et/ou d'oxyde d'éthylène et le composant liquide est stérilisé par filtration.

4. Procédé selon les revendications 1 à 3, **caractérisé en ce qu'**on utilise comme solvant la 2-pyrrolidone, la N-méthylpyrrolidone, la vinylpyrrolidone, le diméthylsulfoxyde, le tétrahydrofuranne, le dioxanne, l'éthylèneglycol, le propanediol ou des associations de ceux-ci.

5. Nécessaire pour la préparation d'un ciment osseux favorisant la libération de substances actives, constitué, dans des conditionnements séparés, de
a) d'un composant solide, dont la proportion va d'environ 50 à 75 % en poids du ciment osseux, essentiellement constitué d'un polymère finement divisé d'esters d'acide acrylique et/ou d'acide méthacrylique et d'une substance active pharmaceutique, et
b) d'une solution d'un composant liquide, dont la proportion va d'environ 25 à 50 % en poids du ciment osseux, essentiellement constitué de monomères d'acide acrylique et/ou d'acide méthacrylique, dans un solvant organique dont la proportion n'excède pas 50 % en poids, par rapport au composant liquide.

6. Nécessaire selon la revendication 5, **caractérisé en ce qu'**il contient un dispositif pour le mélange et/ou l'application du ciment osseux.

7. Nécessaire selon la revendication 5 ou 6, **caractérisé en ce que** l'unité de conditionnement (a) a été soumise à une stérilisation finale au moyen d'irradiation et/ou d'oxyde d'éthylène et le contenu de l'unité de conditionnement (b) a été soumis à une filtration stérilisante.
